(19) Europäisches Patentamt
European Patent Office
Office européen des brevets

(11) EP 2 727 525 A1

(12) **EUROPEAN PATENT APPLICATION**

(43) Date of publication:
**07.05.2014 Bulletin 2014/19**

(51) Int Cl.:
***A61B 5/029*** *(2006.01)* ***A61B 5/00*** *(2006.01)*

(21) Application number: **13189266.3**

(22) Date of filing: **18.10.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.10.2012 JP 2012239185**

(71) Applicant: **Nihon Kohden Corporation**
**Shinjuku-ku**
**Tokyo**
**161-8560 (JP)**

(72) Inventor: **Ogino, Hirokazu**
**Tokyo, 161-8560 (JP)**

(74) Representative: **HOFFMANN EITLE**
**Patent- und Rechtsanwälte**
**Arabellastrasse 4**
**81925 München (DE)**

(54) **Cardiac output measuring unit**

(57) A cardiac output measuring unit includes: a biological signal receiving section which is configured to receive a plurality of biological signals; a calibration information storing section which is configured to store calibration information; a calculation section which is configured to calculate a cardiac output based on the plurality of biological signals and the calibration information; and a communication section which is capable of transmitting the cardiac output to an external device.

*FIG. 1*

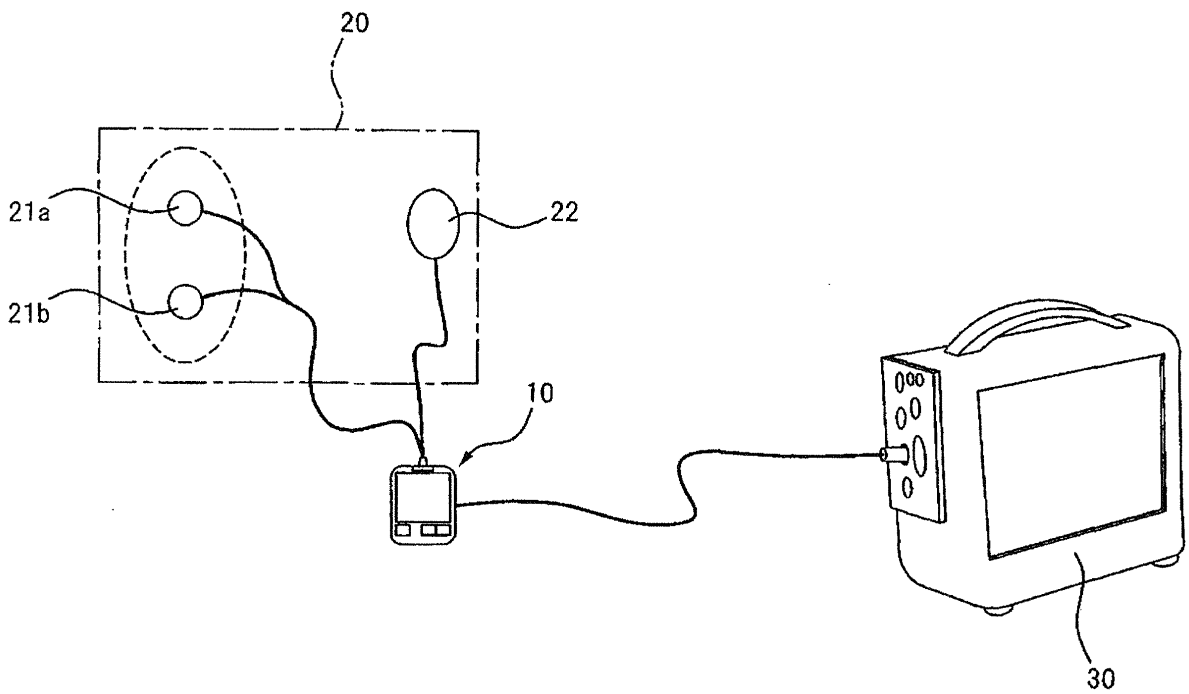


EP 2 727 525 A1

## Description

CROSS-REFERENCE TO RELATED APPLICATION(S)

This application is based upon and claims the benefit of priority from prior Japanese patent application No. 2012-239185, filed on October 30, 2012, the entire contents of which are incorporated herein by reference.

BACKGROUND

The presently disclosed subject matter relates to a cardiac output measuring unit, which can be used by connecting to an existing biological information terminal apparatus such as a bedside monitor.

The applicant of the present application has developed a technique to estimate a cardiac output continuously and non-invasively. The estimated continuous cardiac output (hereinafter, referred to as an esCCO) is obtained by the following equation assuming a cardiac output to be CO, a heart rate to be HR, a pulse wave propagation time to be a PWTT, and patient-specific coefficients to be $\alpha$, $\beta$, and K (refer to JP-A-2005-312947).

$$CO=(\alpha K*PWTT+\beta K)*HR \qquad \text{Equation (1)}$$

In the following explanation, a PWTT is not limited to a pulse wave propagation time, and may include a pulse wave propagation time corresponding to its reciprocal.

The cardiac output measuring apparatus is provided with a calculation function for an esCCO with a blood pressure measuring apparatus connected to a main body side, and is configured to measure a cardiac output by receiving an electrocardiographic waveform transmitted from a telemeter and measurement result information of a blood oxygen saturation.

The cardiac output measuring apparatus resembles a bedside monitor in external form, but is internally configured with hardware and software dedicated to an esCCO. Therefore, to provide a bedside monitor with a function of the cardiac output measuring apparatus, drastic modifications are required, and it is practically impossible.

It is also possible to connect an output of the cardiac output measuring apparatus to an existing bedside monitor. However, if the connection is made in a simple way for measuring esCCO additionally, and a sensor for measuring an electrocardiogram or a pulse wave is attached to a patient, a similar type of the sensor would be attached to a patient. This turns out to be a burden for a patient, or disturbing the work carried out by an operator. Further, in this circumstance, calibration information is required for measuring an esCCO, however, information might be incorrectly entered by mistake. In such a case, an appropriate esCCO may not be output.

SUMMARY

The presently disclosed subject matter may provide a cardiac output measuring unit, which is simple in configuration, and is configured to appropriately measure a cardiac output by connecting it to an existing biological information terminal apparatus.

The cardiac output measuring unit may comprise: a biological signal receiving section which is configured to receive a plurality of biological signals; a calibration information storing section which is configured to store calibration information; a calculation section which is configured to calculate a cardiac output based on the plurality of biological signals and the calibration information; and a communication section which is capable of transmitting the cardiac output to an external device.

The communication section may be capable of receiving the calibration information from the external device, and the calibration information storing section may store the received calibration information.

The cardiac output measuring unit may further comprise: an input section which enables a user to input the calibration information. The calibration information storing section may store the input calibration information.

The biological signal receiving section may receive the plurality of biological signals transmitted from the external device.

The cardiac output measuring unit may further comprise: a biological signal measuring section which is configured to measure the plurality of biological signals and transmit the plurality of biological signals to the biological signal receiving section.

The biological signal measuring section may include: at least two electrodes to measure an R-wave of an electrocardiographic waveform; and a photoelectric pulse wave detection sensor, and the calculation section may calculate the cardiac output by using a pulse wave propagation time.

The at least two electrodes and the photoelectric pulse wave detection sensor may be integrally formed.

The calibration information may include one of a patient's height, weight, body-surface area, gender, blood pressure value at a predetermined time, pulse pressure at a predetermined time, pulse wave propagation time at a predetermined time, and pulse wave at a predetermined time.

The cardiac output measuring unit may further comprise: a display section which is capable of displaying one or more of the cardiac output, the calibration information, and the biological signal.

BRIEF DESCRIPTION OF THE DRAWINGS

Fig. 1 is a configuration diagram of an embodiment of a cardiac output measuring unit according to the presently disclosed subject matter.

Fig. 2 is a block diagram showing an internal configuration of an embodiment of a cardiac output measuring unit according to the presently disclosed subject matter.

DETAILED DESCRIPTION OF EXEMPLARY EMBODIMENTS

Hereinafter, an embodiment of a cardiac output measuring unit according to the presently disclosed subject matter will be explained with reference to accompanying drawings. In each drawing, the same reference numerals are given to the same components, and duplicated description would be omitted. Fig. 1 shows a configuration diagram of an embodiment of a cardiac output measuring unit according to the presently disclosed subject matter. In the same drawing, a reference numeral 10 denotes a main body of a cardiac output measuring unit, to which a sensor 20 is connected. The main body 10 is connected to a bed-side monitor 30, which is an external device constituting a biological information terminal apparatus, in such a way that transmission and reception in-between can be made wirelessly or through a wired line.

The sensor 20 comprises at least two electrodes 21a and 21b to measure an R-wave of an electrocardiographic waveform, and a photoelectric pulse wave detection sensor 22. Two electrodes 21a and 21b may be formed in a single unit. The photoelectric pulse wave detection sensor 22 can be comprised of a pulse oximeter, however, it may be also comprised of a sensor which detect either red (R) light or infrared (IR) light. By providing such a sensor 20, it becomes unnecessary to provide a configuration for measuring biological information overlapping with an electrocardiograph or a pulse oximeter connected to the bedside monitor 30. Further, the sensor 20 is not necessarily an essential configuration in the presently disclosed subject matter. For example, when the bedside monitor 30 can measure an electrocardiographic waveform and a photoelectric pulse wave, the main body 10 may be configured to receive an electrocardiographic waveform and a photoelectric pulse wave from the bedside monitor 30 by a biological signal receiving section to be described later.

Fig. 2 is a block diagram of a cardiac output measuring unit showing an internal configuration of the main body 10. A signal R detected by two electrodes 21a and 21b is guided to an R-wave measuring section 23, and is considered to be a signal indicating the occurrence time of an R-wave of an electrocardiogram. The signal is digitized by an analog-digital (A/D) converter 24, and is sent to a processing section 1. The processing section 1 includes a biological signal receiving section 18 to receive a plurality of biological signals, for instance, a signal indicating the occurrence time of an R-wave of an electrocardiogram. The signal is further sent to a calculation section 11, and the calculation section 11 can obtain a heart rate HR.

A signal detected by the photoelectric pulse wave detection sensor 22 is taken into a pulse wave detection section 25. Thereby, the pulse wave detection section 25 detects a pulse wave of a part of a patient body to which the photoelectric pulse wave detection sensor 22 is attached. An output of the pulse wave detection section 25 is digitized by an analog-digital (A/D) converter 26, and is sent to the biological signal receiving section 18. Receiving the signal, the biological signal receiving section 18 sends the signal to the calculation section 11, and the calculation section 11 can obtain a pulse wave propagation time (PWTT) based on the signal from the R-wave measuring section 23 and the signal from the photoelectric pulse wave detection sensor 22. In the configuration, the electrodes 21a and 21b, photoelectric pulse wave detection sensor 22, R-wave measuring section 23, and pulse wave detection section 25 constitute a biological signal measuring section 2. The biological signal measuring section 2 sends these measurement results to the biological signal receiving section 18.

A display section 13 is connected to the processing section 1 via a display control section 12. The display section 13 may display a message concerning an operation guide, as well as displaying a cardiac output, calibration information, and patient information. An input section 15 is connected to the processing section 1 via an input control section 14. The input section 15 can be comprised of a keyboard or a touch panel, and enables a user to input necessary information such as calibration information. Further, a communication section 16 is connected to the processing section 1. The communication section 16 is connected to the bedside monitor 30 wirelessly or via a wired line, which is a configuration to perform transmission and reception of information.

The processing section 1 includes a calibration information storing section 19 to store calibration information. Calibration information is required to calculate an esCCO, and this is information other than a biological signal received by the biological signal receiving section 18. In particular, calibration information includes any one of patient information such as $\alpha$, $\beta$, and K (e.g. patient-specific coefficients) to be explained later, information such as a CO value for calibration at a predetermined time, and information such as a patient's height, weight, body-surface area, gender, pulse pressure at a predetermined time, heart rate at a predetermined time, pulse wave propagation time at a predetermined time, and/or pulse wave at a predetermined time. Calibration information is obtained from the information stored in the bedside monitor 30, which is an external device communicated by the communication section 16, and the calibration information is stored in the calibration information storing section 19. A user, who may be a medical worker, can input calibration information from the input section 15. The calibration information storing section 19 can store the input calibration information.

The calculation section 11 is configured to calculate a cardiac output by using a heart rate assumed to be HR, a pulse wave propagation time assumed to be a PWTT, and calibration information including patient information. For further details of calculation method of esCCO, it is desirable to use a method as described in JP-A-2005-312947 or JP-A-2010-246801.

The calculation section 11 calculates a cardiac output by the following equation.

$$CO=(\alpha K*PWTT+\beta K)*HR \qquad \text{Equation (1)}$$

Where, a cardiac output is assumed to be CO, a heart rate is assumed to be HR, a pulse wave propagation time is assumed to be a PWTT, and patient information (patient-specific coefficients) is assumed to be $\alpha$, $\beta$, and K. For a patient-specific coefficient $\alpha$, a CO value for calibration, and blood information as calibration information, those stored by the calibration information storing section 19 can be used.

Further, the calculation section 11 may transmit a cardiac output obtained by itself to the bedside monitor 30 via the communication section 16, so that the bedside monitor 30 displays the cardiac output. A cardiac output obtained by the calculation section 11 may also be sent to the display control section 12, so that the cardiac output is displayed on the display section 13 under its control.

In the above description, a cardiac output is obtained by a biological signal obtained from the biological signal measuring section 2. However, as an alternative aspect, the biological signal receiving section 18 may receive a plurality of biological signals transmitted from the bedside monitor 30, which is an external device, via the communication section 16 so that a cardiac output may also be obtained by using these biological signals.

According to an aspect of the presently disclosed subject matter, the cardiac output measuring unit can output a cardiac output from an existing biological information terminal apparatus without largely modifying the biological information terminal apparatus. Further, the cardiac output measuring unit is simple in its configuration, and is configured to appropriately measure a cardiac output without increasing the workload of an operator for medical treatment, or placing a burden on a patient. Moreover, since a cardiac output can be calculated by using biological signals and calibration information, which can be obtained from the existing biological information terminal apparatus, it is possible to provide a medical worker with useful information from the existing data.

## Claims

1. A cardiac output measuring unit comprising:

   a biological signal receiving section which is configured to receive a plurality of biological signals;
   a calibration information storing section which is configured to store calibration information;
   a calculation section which is configured to calculate a cardiac output based on the plurality of biological signals and the calibration information; and
   a communication section which is capable of transmitting the cardiac output to an external device.

2. The cardiac output measuring unit according to claim 1, wherein
   the communication section is capable of receiving the calibration information from the external device, and
   the calibration information storing section stores the received calibration information.

**3.** The cardiac output measuring unit according to claim 1 or 2, further comprising:

an input section which enables a user to input the calibration information, wherein
the calibration information storing section stores the input calibration information.

**4.** The cardiac output measuring unit according to any one of claims 1 to 3, wherein
the biological signal receiving section receives the plurality of biological signals transmitted from the external device.

**5.** The cardiac output measuring unit according to any one of claims 1 to 4, further comprising:

a biological signal measuring section which is configured to measure the plurality of biological signals and transmit the plurality of biological signals to the biological signal receiving section.

**6.** The cardiac output measuring unit according to claim 5, wherein
the biological signal measuring section includes: at least two electrodes to measure an R-wave of an electrocardiographic waveform; and a photoelectric pulse wave detection sensor, and
the calculation section calculates the cardiac output by using a pulse wave propagation time.

**7.** The cardiac output measuring unit according to claim 6,
wherein
the at least two electrodes and the photoelectric pulse wave detection sensor are integrally formed.

**8.** The cardiac output measuring unit according to any one of claims 1 to 7, wherein
the calibration information includes one of a patient's height, weight, body-surface area, gender, blood pressure value at a predetermined time, pulse pressure at a predetermined time, pulse wave propagation time at a predetermined time, and pulse wave at a predetermined time.

**9.** The cardiac output measuring unit according to any one of claims 1 to 8, further comprising:

a display section which is capable of displaying one or more of the cardiac output, the calibration information, and the biological signal.

FIG. 1

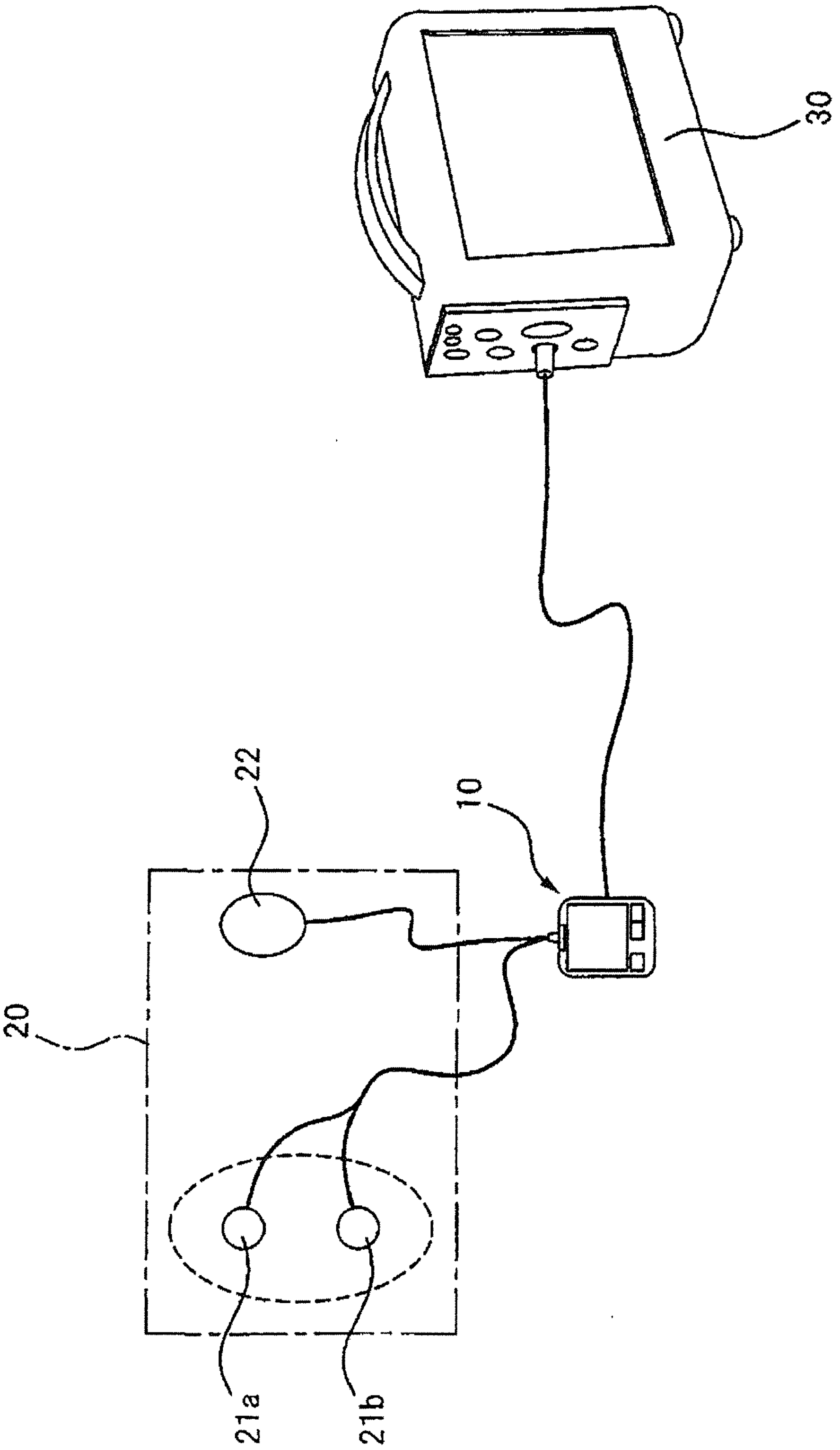

FIG. 2

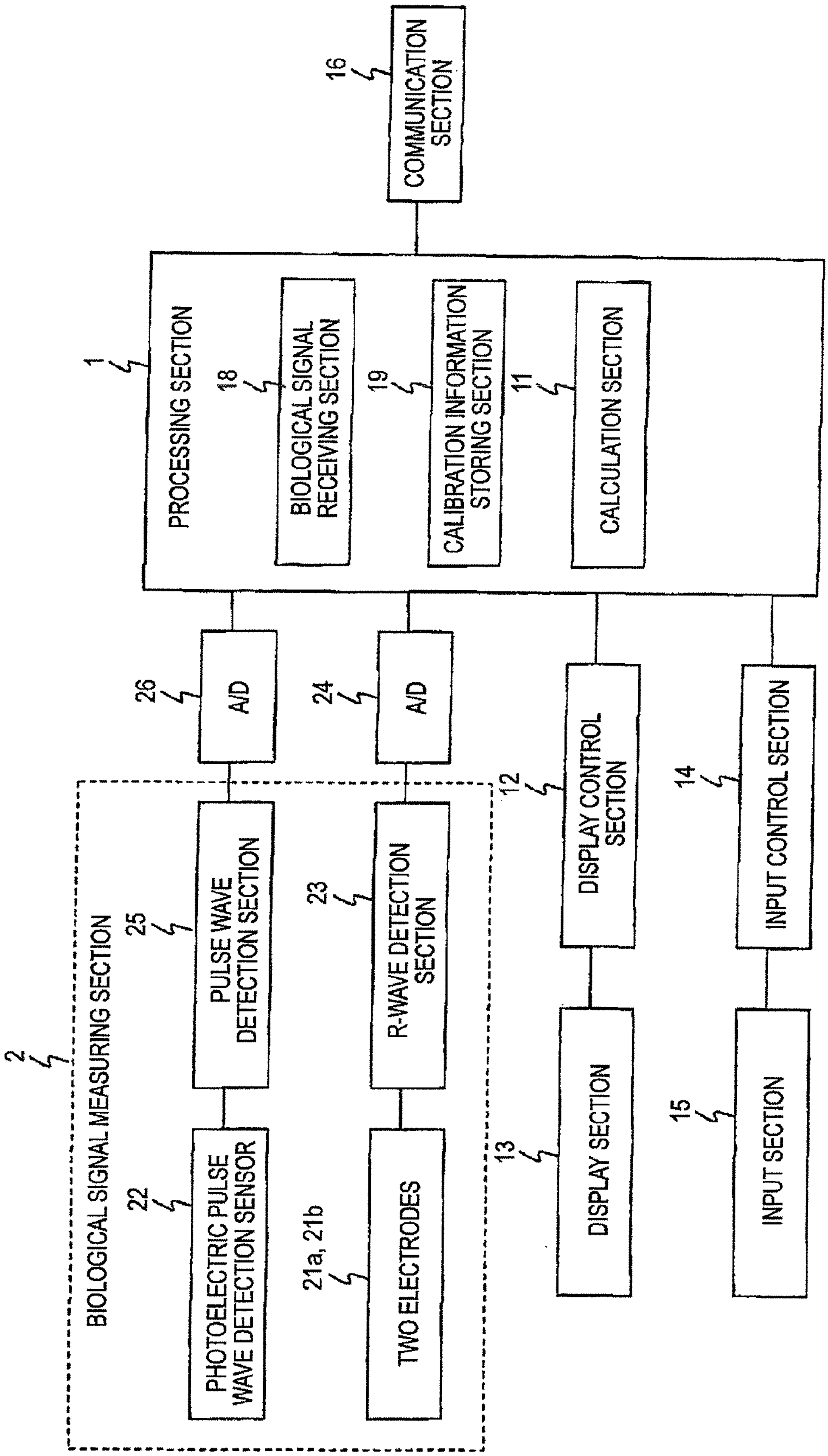
COMMUNICATION SECTION
16
1
PROCESSING SECTION
18
BIOLOGICAL SIGNAL RECEIVING SECTION
19
CALIBRATION INFORMATION STORING SECTION
11
CALCULATION SECTION
26
A/D
24
A/D
12
DISPLAY CONTROL SECTION
14
INPUT CONTROL SECTION
2
BIOLOGICAL SIGNAL MEASURING SECTION
25
PULSE WAVE DETECTION SECTION
23
R-WAVE DETECTION SECTION
22
PHOTOELECTRIC PULSE WAVE DETECTION SENSOR
21a, 21b
TWO ELECTRODES
13
DISPLAY SECTION
15
INPUT SECTION

Europäisches Patentamt
European Patent Office
Office européen des brevets

# EUROPEAN SEARCH REPORT

Application Number
EP 13 18 9266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2005/222514 A1 (SUGO YOSHIHIRO [JP] ET AL) 6 October 2005 (2005-10-06)<br>* paragraph [0026] - paragraph [0032] *<br>* paragraph [0056] - paragraph [0059] *<br>* paragraph [0070] - paragraph [0074] *<br>* paragraph [0076] - paragraph [0082] *<br>* paragraph [0096] *<br>* figures 7,8 *<br>----- | 1,5-9<br><br>2-4 | INV.<br>A61B5/029<br>A61B5/00 |
| Y | US 2005/124866 A1 (ELAZ JOSEPH [US] ET AL) 9 June 2005 (2005-06-09)<br>* paragraph [0002] *<br>* paragraph [0004] *<br>* paragraph [0015] *<br>* paragraph [0022] - paragraph [0026] *<br>* paragraph [0029] *<br>* paragraph [0043] *<br>* figure 2 *<br>----- | 2-4 | |
| A | US 2004/147818 A1 (LEVY ANDREW [US] ET AL) 29 July 2004 (2004-07-29)<br>* paragraph [0011] *<br>* paragraph [0013] *<br>* paragraph [0015] *<br>* paragraph [0023] - paragraph [0024] *<br>* paragraph [0027] *<br>* paragraph [0033] *<br>* paragraph [0035] *<br>* paragraph [0039] *<br>* paragraph [0041] *<br>* figures 1-5 *<br>-----<br>-/-- | 1 | TECHNICAL FIELDS SEARCHED (IPC)<br>A61B |

The present search report has been drawn up for all claims

1

| Place of search | Date of completion of the search | Examiner |
|---|---|---|
| Berlin | 8 January 2014 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 18 9266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/120164 A1 (NIELSEN ERIC [US] ET AL) 26 June 2003 (2003-06-26)<br>* paragraph [0005] - paragraph [0007] *<br>* paragraph [0028] - paragraph [0029] *<br>* paragraph [0031] *<br>* paragraph [0036] *<br>* paragraph [0039] *<br>* paragraph [0052] *<br>* paragraph [0068] - paragraph [0069] *<br>* paragraph [0079] - paragraph [0083] *<br>* figures 1,20A,20B,21 *<br>----- | 1 | |
| A | US 2010/268101 A1 (SUGO YOSHIHIRO [JP]) 21 October 2010 (2010-10-21)<br>* paragraph [0068] - paragraph [0078] *<br>* paragraph [0113] - paragraph [0117] *<br>* figure 1 *<br>----- | 1,5-9 | |

TECHNICAL FIELDS SEARCHED (IPC)

The present search report has been drawn up for all claims

1

| Place of search | Date of completion of the search | Examiner |
|---|---|---|
| Berlin | 8 January 2014 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 9266

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2014

| Patent document cited in search report | Publication date | Patent family member(s) | Publication date |
|---|---|---|---|
| US 2005222514 A1 | 06-10-2005 | US 2005222514 A1 | 06-10-2005 |
| | | US 2007167852 A1 | 19-07-2007 |
| US 2005124866 A1 | 09-06-2005 | CN 1871610 A | 29-11-2006 |
| | | EP 1690209 A1 | 16-08-2006 |
| | | JP 2007510504 A | 26-04-2007 |
| | | US 2005124866 A1 | 09-06-2005 |
| | | US 2008000479 A1 | 03-01-2008 |
| | | WO 2005050525 A1 | 02-06-2005 |
| US 2004147818 A1 | 29-07-2004 | AU 2003293379 A1 | 29-06-2005 |
| | | US 2004147818 A1 | 29-07-2004 |
| | | WO 2005055822 A1 | 23-06-2005 |
| US 2003120164 A1 | 26-06-2003 | DE 10259780 A1 | 03-07-2003 |
| | | FR 2834628 A1 | 18-07-2003 |
| | | JP 4280806 B2 | 17-06-2009 |
| | | JP 2003220045 A | 05-08-2003 |
| | | US 2003120164 A1 | 26-06-2003 |
| US 2010268101 A1 | 21-10-2010 | EP 2241251 A1 | 20-10-2010 |
| | | JP 5330069 B2 | 30-10-2013 |
| | | JP 2010246801 A | 04-11-2010 |
| | | US 2010268101 A1 | 21-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012239185 A
- JP 2005312947 A
- JP 2010246801 A